Europäisches Patentamt

European Patent Office

Office européen des brevets

(11) Publication number: **0 213 811**
A2

(12)

# EUROPEAN PATENT APPLICATION

(21) Application number: 86306115.6

(22) Date of filing: 07.08.86

(51) Int. Cl.⁴: **A 61 K 47/00**
A 61 K 35/74, A 61 K 49/00
A 61 K 43/00

(30) Priority: 16.08.85 US 766360

(43) Date of publication of application:
11.03.87 Bulletin 87/11

(84) Designated Contracting States:
AT BE CH DE FR GB IT LI LU NL SE

(71) Applicant: JOHNSON MATTHEY, INC.,
4 Malin Road
Malvern Pennsylvania 19355(US)

(72) Inventor: Picker, Donald H.
310 Woodside Avenue
Narberth Pennsylvania 19072(US)

(72) Inventor: Serino, Anthony, Jr.
1143 Pynchon Hall Road
West Chester Pennsylvania 19382(US)

(72) Inventor: Henson, Geoffrey W.
Blackhawk M-112
Downingtown Pennsylvania 19335(US)

(74) Representative: Arthur, Bryan Edward et al,
Withers & Rogers 4 Dyer's Buildings Holborn
London EC1N 2JT(GB)

(54) Bateriocin-Targeted compounds for cancer therapy.

(57) Compositions comprising a bacteriocin or active fragment thereof linked directly or indirectly to an antitumor compound, radioisotope, boron-10 containing compound, radiosensitizer, or other therapeutic or analytical agent.

BACTERIOCIN-TARGETED COMPOUNDS FOR CANCER THERAPY

This invention relates to novel bacteriocin-conjugates for the detection, localization, and/or treatment of tumor cells and tissues.

BACKGROUND OF THE INVENTION

The use of a variety of organic and inorganic compounds in the chemotherapeutic treatment of cancer is now an established clinical technique. Nonetheless, efforts continue to find and develop new and improved compounds. The problem with most compounds when administered as a composition together with an inert carrier or diluent is that they are absorbed generally into the systemic circulation from where they have a toxic effect on normal cells and tissues as well as on diseased cells and tissue which they are designed to treat. In practice, the maximum dose that can be administered is limited not by pharmaceutical effectiveness but by toxicity, with the result that the patient suffers unpleasant or even severe side effects.

In an attempt to render antitumor compounds specific and less toxic for certain types of tumor cells, many investigators have linked these antitumor compounds to carriers, with varying therapeutic success, such as DNA, liposomes, antibodies, hormones and various proteins. See Rowland, G.F., (1983) Clinics in Immunol. & Allergy, 3, 235-257; Trouet, A. (1972) Nature, New Biol. 239, 110-112; Rustum, Y.M. (1979) Cancer Res. 39, 1390-1395; Ghose, T. (1978) J. Natl. Cancer Inst. 61, 657-677; Kaneko, Y. (1981) Horm. Met. Res. 13, 110-114; and Ryser, H.J.P. (1978) Proc. Natl. Acad. Sci. 75,3867-3870.

## OBJECTS OF THE INVENTION

It has been demonstrated that a variety of bacteriocins recognize tumor cells but do not recognize normal cells. See: Farkas-Himsley, H. (1983) IRCS Med. Sci. 11, 236-237. It is an object of this invention, therefore, to target a large variety of antitumor compounds to tumor cells and tissues by the covalent and/or ionic attachment of such compounds to bacteriocins or active fragments thereof, or bacterial proteinaceous products. See Farkas-Himsley, H. (1976) Cancer Res. 36, 3561-3567.

A further object of this invention is to provide bacteriocin-antitumor conjugates that are stable in vivo and which will release enough of the active antitumor compound at the target site to achieve a therapeutic effect.

A further object of this invention is to provide bacteriocin-conjugates of radioisotopes which will allow for the detection and localization of a tumor mass or metastastes.

A further object of this invention is to provide bacteriocin-conjugates of boron-10 containing compounds which can be used for tumor therapy in conjunction with thermal neutron irradiation.

A further object of this invention is to provide bacteriocin-conjugates of radiosensitizers which can be used for tumor therapy in conjunction with ionizing radiation.

Upon examination of the specifications and appended claims, further objects and advantages of this invention will be apparent to those skilled in the art.

## DESCRIPTION OF THE INVENTION

Accordingly, the bacteriocin or active fragment may be linked to the antitumor compound directly or indirectly through a spacer which can be a degradable or non-degradable linker or biopolymer. As will be appreciated, the bacteriocin delivers the antitumor compound to the target site where either the antitumor compound is liberated, or the conjugate is internalized, for therapeutic use.

Any of the available bacteriocins which have the characteristic of selectively recognizing tumor cells may be used for present purposes but this can include active fragments as well. Typical examples include Vibriocin 41-S, Vibriocin 506, Pyocin P-1, Pyocin P-4, Colicin HSC 10 Mycobacteriocin, Colicin HSC 4, etc. which are formed from bacteria such as Vibrio Cholerae, Vibrio eltor, Echerichia coli, Pseudomonas aeruginosa and Pseudomonas pyocyanea. See Farkas-Himsley and Musclow (1985) submitted to Cell. Mol. Biol.

The antitumor compound can be any of the known antineoplastic agents. Representative examples of such compounds for use herein include Daunomycin, Adriamycin, Mitomycin-C, Methotrexate, cis-Platin, Chlorambucil, Trenimon, Phenylene diamine mustard, Bleomycin, Vindesine, Daunorubin, 5 Fluorouridine, Cytosine arabinoside, Neocarzinostatin, Vincristine, Etoposide, Cyclophosphamide, Phospholipase C, Glucose Oxidase,

4

or any other antitumor compound, including functionalized active derivatives.

The bacteriocin or active fragment and the antitumor compound may be linked directly or indirectly utilizing conventional techniques provided that these do not significantly alter the characteristic properties of the two components. The linkage can involve either covalent or ionic attachment brought about by the appropriate chemical reaction. The attachment will usually involve linkage through carboxyl groups, amino groups, sulfhydryl groups, or sugar residues on any of the reacting species. A spacer molecule, which may be cleavable or non-cleavable, can be advantageously employed. Such spacers includes, among others, the aconityl molecule, protein modification reagents, e.g. glutaraldehyde, and polypeptides. Additionally a carrier, e.g. dextran or a biopolymer such as poly-glutamic acid or a protein or protein fragment, can be modified by the attachment of the antitumor compound and then attached, either directly or indirectly and with or without a spacer molecule as described above, to the bacteriocin or active fragment. The reaction conditions for the above described attachments are such that (i) the binding specificity of the bacteriocin is retained (i.e. that the bacteriocin or active fragment is able to recognize tumor cell surface receptors and not recognize normal cells), (ii) that the bacteriocin-conjugate remains soluble under physiological conditions, and (iii) that the bacteriocin-conjugate can be localized in vivo.

Among the radioisotopes which may be used to radiolabel bacteriocins or active fragments for the detection and localization of a tumor mass or metastases are gamma emitters, positron emitters, x-ray emitters, and fluorescence emitters. A preferred method of radiolabeling, however, utilizes either Iodine-131 or Iodine-125 in an oxidative procedure such as reported by Greenwood, et al (1963) Biochem. J. 89, 114, and later modified by McConahey, et al (1969) Int. Arch. Allergy Appln. Immunol. 29, 185. Isotopes other than those of Iodine which can be detected by gamma cameras and which may be utilized above include Gallium-67, Technetium-99m, and Indium-111. See: Khaw, et al (1980) Science 209, 295; Scheinberg, et al (1982) Science 215, 1511; Hantowich, et al (1982) Int. J. Appl. Radiat. Isot. 33, 327; Crockford, et al (198?) U.S. Pat. No. 4,323,546; and Wong, et al (1978) Int. J. Appl. Radiat. Isot. 29, 251.

Bacteriocin-conjugates of Boron-10 containing compounds of at least the 19.6% natural abundance of the Boron-10 isotope for use in a Neutron-Capture Therapy of tumor tissue may be prepared and utilized according to the teachings of the invention. The Boron-10 containing compound can be any of the known boron compounds but is preferably a cluster molecule bearing a functionality suitable for coupling to protein or carrier molecules by any of a variety of well-documented procedures. See: Lipscomb, W.N., et al (1974) J. Med. Chem. 17, 785; Lipscomb, W.N., et al (1974) J. Med. Chem. 17, 792.

Additionally, the bacteriocin-boron-10 conjugate may be radiolabeled by one or more of the well known procedures for radiolabeling proteins, as described above, or by incorporating the radiolabel into the boron cluster by chemical synthesis prior to coupling, for the simultaneous or consecutive localization and therapy of tumor tissue. See: Hawthorne, M.F., et al (1985) Inorg. Chem. 24, 1911-1916.

It has been shown that certain organic and inorganic compounds are effective radiosensitizers in hypoxic cells. Representative examples include metronidazole, misonidazole, and cis-Platin. See: Double and Richmond (1978) Br. J. Cancer 37 (Suppl. III) 98-102; and Adams, et al (1976) Radiat. Res. 67, 9-20; Chibber, et al (1984) Int. J. Radiation Oncology Biol. Phys. 10, 1213-1215; and Coughlin, et al (1985) Int. J. Radiation Oncology Biol. Phys. 11, 915-919. Bacteriocin-conjugates of radiosensitizers may be prepared and utilized according to the teachings of the invention. The radiosensitizer may be any of the known organic, inorganic, or organometallic compounds useful in the treatment of hypoxic tumors in conjunction with ionizing radiation, including functionalized active derivatives.

The invention is illustrated by the following examples:

## Example 1
### Preparation of the bacteriocin

The bacteriocins were prepared and titrated as previously described for Vibriocin by Jayawardene and Farkas-Himsley (1969) Microbios, 4, 325-333. Briefly, the cultures were induced with Mitomycin-C, incubated and then resuspended in 0.5% NaCl solution at pH 7.8. Upon further incubation, a cell lysate in the NaCl solution containing the bacteriocin was obtained. The cell debris was then removed by centrifugation and the bacteriocin-containing supernatant was concentrated by filtration with a PM 10 membrane, and sterilized with the use of Millipore filters (0.45 µm pore size). Further purification is done by the method of Farkas-Himsley and Yu (1985) Cytobios. 42, 193-207. Briefly, this involves an ammonium sulfate precipitation of the filtered lysate, followed by resuspension in 30 mM Tris buffer (pH 7.5), chromatography on Sephadex-A50 using a gradient of 0.1 M to 0.4 M with the purified fraction eluting in the vicinity of 0.2 M Tris.HCl.

## Example 2

The bacteriocin of example 1 was linked directly to an antitumor compound as follows:

To 0.1 ml of Diaminecarboxypentyl-malonatoplatinum (II) in dimethyladetamide (DMA) at a concentration of 120 mg/ml was added 0.1 ml of triethylamine in DMA (at a concentration of 0.037 ml/ml). After cooling to 4°C. 0.1 ml of isobutylchloroformate in DMA (at a concentration of 0.035 ml/ml) was added, stirred 1 hour, then added in one portion to 2.7 ml of the bacteriocin in 0.1 M phosphate, pH 8.6 (at a concentration of 5.3 mg/ml). The reaction was stirred overnight then desalted on Sephadex G25 and dialyzed extensively against water. Further purification can be achieved using gel filtration media, such as LKB AcA 54. The appropriate fractions are pooled, assayed, and lyophilized. Platinum content is quantified by either an ICP or graphite furnace AA analysis and protein content is determined by the Bradford dye-binding assay.

## Example 3

The bacteriocin of example 1 was linked to a boron-10 containing compound through a carrier molecule as follows:

Dextran (average molecular weight = 40000) is oxidized by a 30-fold molar excess of sodium meta-periodate in 100mM sodium acetate at pH 5.6 for 16 hours. The oxidized dextran is dialyzed against distilled water and lyophilized, then reacted with a 25-fold molar excess of C-Amminecarbaundecaborane at pH 6.5 in distilled water for 6 hours. The resulting Schiff bases are reduced with an equimolar amount of sodium cyanoborohydride at pH 6.0 for 3 hours, and the product dialyzed against distilled water and lyophilized. Subsequent reaction with the bacteriocin at a molar ratio of 10:1 is performed in phosphate-buffered saline (PBS) at pH 6.5 for 6 hours at 4°C, followed by reduction by a 10% molar excess of sodium cyanoborohydride at pH 6.5 for 3 hours at 4°C, and dialysis against PBS, pH 7.4. Boron content was quantified by both ICP and prompt-gamma analyses, and protein concentration determined by the Bradford dye-binding assay.

## Example 4

The bacteriocin of example 1 was linked to an antitumor compound through a spacer molecule as follows:

Daunomycin hydrochloride, 1.0 ml (at a concentration of 5 mg/ml) in water was basified to pH 8.5 at 4°C by the dropwise addition of 0.1N sodium hydroxide. An equimolar amount of cis-aconitic anhydride was added while maintaining the pH at 8.5. The solution was stirred 5 minutes at 4°C, then 1N hydrochloric acid was added dropwise to form a precipitate which was collected by centrifugation. The pellet was resuspended in 0.5 ml phosphate-buffered saline (PBS) at pH 7.3 and added as a 30-fold molar excess to the bacteriocin in PBS (at a concentration of 5 mg/ml). A 50-fold molar excess of 1-ethyl-3-(3-dimethylaminopropyl)carbodiimide in PBS was then added and the mixture stirred 16 hours while protected from light. The bacteriocin-conjugate was desalted on Sephadex G25 then dialyzed against distilled water. Drug-bacteriocin ratios were determined by measuring the concentration of duanomycin by its absorption at 495 nm (E=10000) and bacteriocin concentration by the Bradford dye-binding assay.

Any one of the bacteriocin-conjugates described in this invention can be used in a variety of pharmaceutical formulations and can be administered by a variety of conventional routes, such as intramuscular, intravenous, subcutaneous, and intraperitoneal. When administering the bacteriocin-parenterally, pharmaceutically-acceptable forms for injection may include sterile aqueous solutions of dispersions, and sterile powders for reconstitution into sterile injectable solutions or dispersions. Various antibacterial and antifungal agents, e.g. parabens, phenol, sorbic acid, chlorobutanol, and the like may be used. Sugars, sodium chloride, and isotonic agents may also be desirable additives. Prolonged absorption of the injectable dose may be accomplished by agents which delay absorption, e.g. aluminum monostearate and gelatin. Appropriate doses of the bacteriocin-conjugate are administered to achieve the desired diagnostic and/or therapeutic effect.

In the testing of Bacteriocin-conjugates, verification of binding and activity of the bacteriocin-conjugate can be performed by one or more of the following assays:

1. Tritiated-thymidine uptake inhibition. See: Farkas-Himsley (1980) Microbios. Lett. 15, 89-96; and Farkas-Himsley and Musclow (1980) IRCS Med. Sci. 8, 497-498.

2. Fluorescence assay. See: Farkas-Himsley and Musclow (1985) Cell. Molec. Biol., submitted.

3. Iodine-125 assay. Farkas-Himsley, personal communication.

4. Flow cytometry. Farkas-Himsley and Musclow (1980) Cell. and Mol. Biol. 26, 597-603.

5. Cell survival by counting. Farkas-Himsley and Cheung (1976) Cancer Res. 36, 3561-3567.

It will be appreciated by those skilled in the art that various other modifications are contemplated according to and implied by the invention. Accordingly, the scope of the invention is defined in the following claims wherein:

CLAIMS:

1. A composition comprising a bacteriocin or active fragment linked to an agent selected from the group consisting of antitumor compound, radioisotope, boron-10 containing compound, radiosensitizer, or other therapeutic or analytical agent.

2. A composition according to claim 1 wherein the bacteriocin or active fragment and the agent are linked directly together by means of a covalent or ionic bond.

3. A composition according to claim 1 wherein the bacteriocin or active fragment and the agent are linked together through a spacer and/or a carrier molecule, either of which may be cleavable or non-cleavable in vivo.

4. A composition according to claim 1 wherein the agent is an anti-tumor compound.

5. A method of detecting, localizing, and/or treating a tumor or metastatic cells which comprises administering a composition according to claim 1.